# EUROPEAN PATENT APPLICATION

(11) **EP 4 702 979 A1**
(43) Date of publication of application: **04.03.2026**
(21) Application number: 24796255.8
(22) Date of filing: 26.04.2024
(51) Int. Cl.: A61K 31/568, A61P 25/00

(54) **USE OF COMPOUND IN PREVENTION OR TREATMENT OF RADIATION-INDUCED BRAIN INJURY**

(30) Priority: 28.04.2023 CN 202310482604
(71) Applicant: Guangzhou Cellprotek Pharmaceutical Co., Ltd, Guangzhou, Guangdong 510663 (CN)
(72) Inventor: HUANG, Yijun, Guangzhou, Guangdong 510663 (CN); CHEN, Yupin, Guangzhou, Guangdong 510663 (CN); YUAN, Mingjun, Guangzhou, Guangdong 510663 (CN); LU, Bingzheng, Guangzhou, Guangdong 510663 (CN); WANG, Yana, Guangzhou, Guangdong 510663 (CN); HUANG, Chunhui, Guangzhou, Guangdong 510663 (CN); HUANG, Jiayu, Guangzhou, Guangdong 510663 (CN); YAN, Guangmei, Guangzhou, Guangdong 510663 (CN)
(74) Representative: J A Kemp LLP
(86) International application number: PCT/CN2024/090100
(87) International publication number: WO 2024/222883

(57) **Abstract**

Disclosed is use of 5α-androst-3β,5,6β-triol and derivatives thereof in the prevention or treatment of radiation-induced brain injury, and a method for preventing or treating radiation-induced brain injury with 5α-androst-3β,5,6β-triol and derivatives thereof.

## Description

### Technical Field

The present disclosure relates to use of 5α-androst-3β,5,6β-triol and derivatives thereof in the prevention or treatment of radiation-induced brain injury.

### Background

Radiotherapy is an important treatment method for primary and metastatic tumors in the head and neck region. Disease with central nervous system damage symptoms develop in patients with head and neck tumors after radiotherapy, and is a serious complication of radiotherapy in cancer patients. The incidence of radiation-induced brain injury (RBI) after stereotactic radiotherapy for meningiomas is 28-50%, the 4-year cumulative incidence of radiation-induced brain injury after radiotherapy for nasopharyngeal carcinoma is 1.9-5%, the 4-year cumulative incidence of radiation-induced brain injury after radiotherapy for poorly differentiated gliomas is 1-24%, and the 1-year cumulative incidence of radiation-induced brain injury after radiotherapy for brain metastases is 8-20%. Among radiation-induced brain injuries with nasopharyngeal carcinoma as the primary disease, 33% of patients eventually develop radiation-induced brain injury in the bilateral temporal lobe. The cumulative radiation dose that brain tissue can tolerate is 50-60 Gy. Beyond this range, the incidence of radiation-induced brain injury increases significantly with the increase of cumulative radiation dose. The 5-year cumulative incidence of radiation-induced brain injury can reach over 20% in patients who have undergone further radiotherapy. In today's world where patient quality of life has become second only to survival rate in evaluating the efficacy of radiation therapy, radiation-induced injury is receiving increasing attention.

Radiation-induced brain injury (RBI) is classified into acute type, early-delayed type, and late-delayed type according to the time of occurrence. Acute radiation-induced brain injury is often a part of multi organ damage in acute radiation syndrome (ARS). Symptoms often occur during radiotherapy or several days to one month after irradiation, mostly manifested as headaches, nausea, vomiting, memory loss, and other symptoms in the early stages of irradiation. Severe cases can rapidly progress to consciousness disorders, orientation disorders, ataxia, and some may experience coma and death within a few days. Early-delayed type often occurs 1-6 months after irradiation, manifested as drowsiness, nausea, vomiting, irritability, memory loss, etc. It can also be manifested as transient fatigue or worsening of local neurological symptoms, including clinical subtypes such as drowsiness syndrome, brainstem encephalitis, and pseudoprogression of tumors. Late-delayed type appears 6 months after the end of irradiation and is the most common clinical type of radiation-induced brain injury, also known as delayed radiation-induced brain injury, commonly seen in patients with brain irradiation doses greater than 50 Gy.

The traditional treatment for radiation-induced brain injury is glucocorticoids, but the treatment of radiation-induced brain injury with glucocorticoids is mostly based on clinical experience, case reports, and retrospective analysis results. Currently, there is still a lack of large-scale clinical randomized controlled trials. A randomized double-blind placebocontrolled study in 2011 showed that patients treated with bevacizumab had varying degrees of reduction in brain injury lesions and significant improvement in neurological function. In the following 10 months of follow-up, only 2 patients experienced recurrence of radiation-induced brain injury. Research reports indicate that mouse nerve growth factor for injection can alleviate damage to the blood-brain barrier and repair microvessels in animals, suggesting that it can promote the recovery of radiation-induced brain injury. In addition, the use of dehydrating drugs is not a routine symptomatic support method for radiation-induced brain injury. It is recommended to use them only for a short period of time in patients with radiation-induced brain injury who experience rapid progression of the disease and whose imaging confirms the presence of acute space occupying effects in the radiation-induced brain injury lesion. The course of treatment should be within 5-7 days.

Currently, there is still an urgent need for drugs targeting radiation-induced brain injury in clinical practice.

### Summary

In one aspect, the present invention provides use of a compound of formula I: 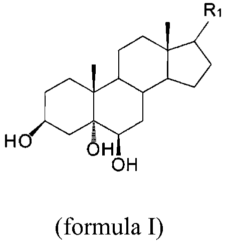
or a pharmaceutically acceptable salt thereof in the manufacture of a medicament for preventing or treating a radiation-induced brain injury,
wherein R₁ is selected from H, -CN, fluoro, chloro, C₁₋₁₀ alkyl, fluoro or chloro substituted C₁₋₁₀ alkyl, C₁₋₁₀ alkoxy, fluoro or chloro substituted C₁₋₁₀ alkoxy, and C₃₋₁₀ cycloalkyl.

In another aspect, the present invention provides a method for preventing or treating a radiation-induced brain injury, comprising administering a compound of formula I or a pharmaceutically acceptable salt thereof to a subject who has undergone a radiation therapy.

In yet another aspect, the present invention provides a compound of formula I or a pharmaceutically acceptable salt thereof for use in preventing or treating a radiation-induced brain injury.

In any one of the above aspects, in some embodiments, the radiation dose for the radiation-induced brain injury is 50 Gy or more.

In any one of the above aspects, in some embodiments, the radiation-induced brain injury is gamma ray radiation-induced brain injury.

In any one of the above aspects, in some embodiments, the radiation-induced brain injury is an early-delayed radiation-induced brain injury or a late-delayed radiation-induced brain injury.

In any one of the above aspects, in some embodiments, the radiation-induced brain injury is the edema stage or necrosis stage of a late-delayed radiation-induced brain injury.

In any one of the above aspects, in some embodiments, R₁ is H, -CHCH₂CH₃, - CH(CH₃)₂, -CH(CH₂)₃CH₃, or -CH(CH₃)(CH₂)₃CH(CH₃)₂, and preferably, R₁ is H.

The compounds provided by the present invention alleviate the damage caused by gamma ray radiation to brain tissue and have good preventive or therapeutic effects on RBI.

### Brief Description of Drawings

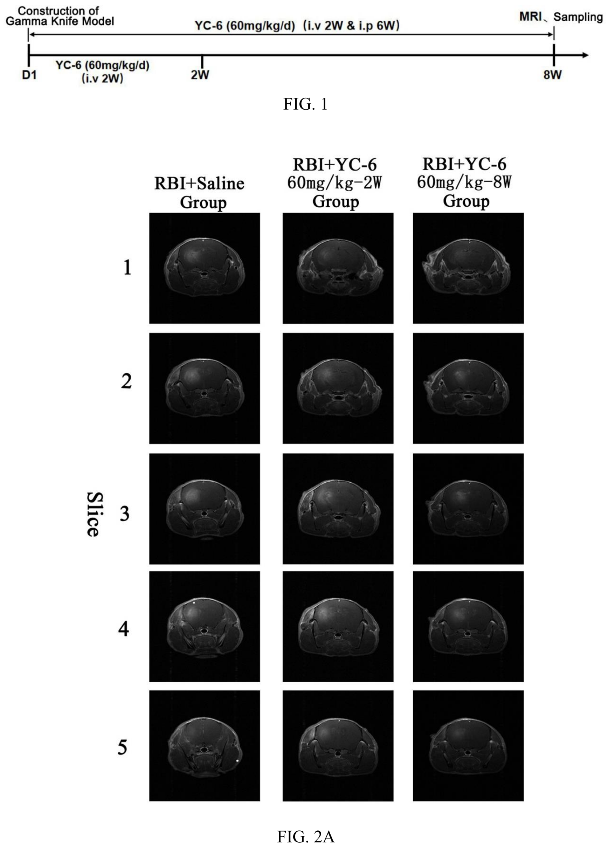

Fig. 1: Experimental flowchart for evaluating the effect of YC-6 in gamma knife irradiation induced focal radiation-induced brain injury in C57BL/6 mice.

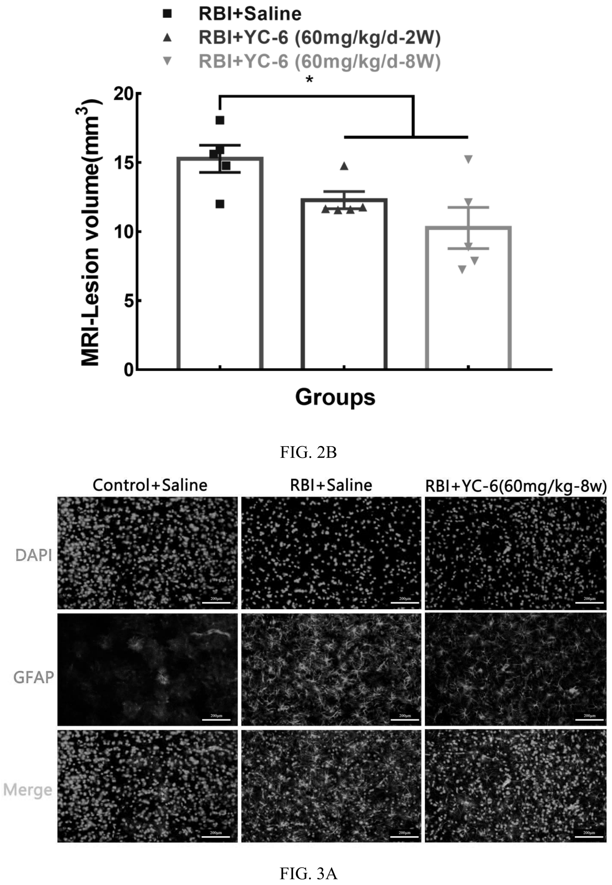

Fig. 2: YC-6 significantly reduced the volume of radiation-induced brain injury induced by gamma ray. Pannel A: Representative MRI images of animal, with 5 brain images in each column representing the brain tissue of each animal; The white signal area represents the damaged lesion area. Pannel B: Reducing the injury caused by gamma ray irradiation to brain tissue. Compared with the RBI+Saline model group, * represents P<0.05.

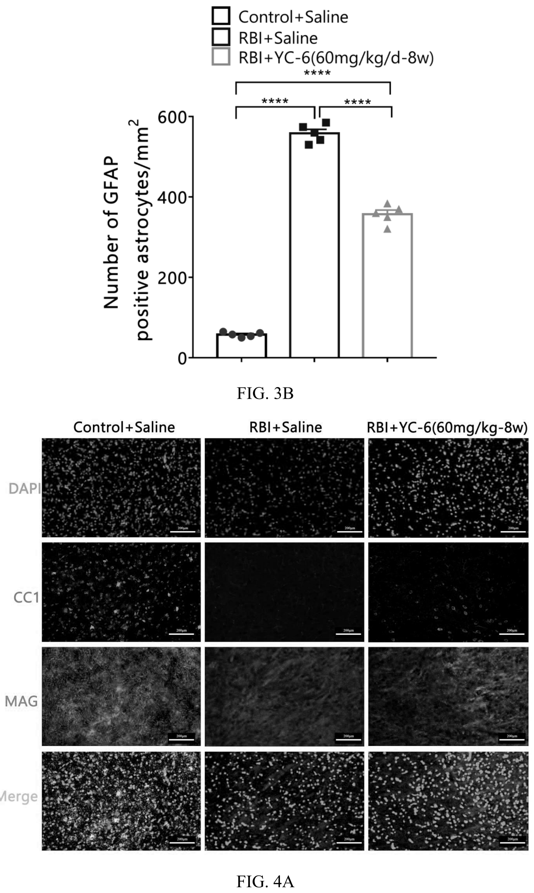

Fig. 3: YC-6 inhibited the activation of astrocytes after radiation-induced brain injury. Pannel A: GFAP immunofluorescence staining in the thalamus of the brain lesion, with a white ruler indicating 200 µm. Pannel B: Counting GFAP positive cells in Pannel A, i.e., randomly selecting three fields of view in the brain slice injury lesion area and using Image J software to count GFAP positive cells in the selected fields as the number of GFAP positive cells per unit area, and taking the average value. **** represents P<0.0001, n=5.

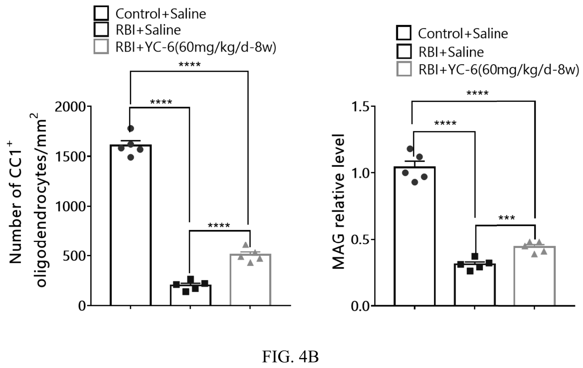

Fig. 4: YC-6 inhibited the reduction of oligodendrocytes and restored MAG level. Pannel A: Immunofluorescence staining of CC1 and MAG in the thalamus of the brain lesion, with a white ruler indicating 200 µm. Pannel B: Counting CC1 positive cells in Pannel A, i.e., randomly selecting three fields of view in the brain slice injury lesion area and using Image J software to count CC1 positive cells in the selected fields as the number of CC1 positive cells per unit area, and taking the average value. Pannel C: Statistical analysis of MAG immunofluorescence intensity in Pannel A, i.e., randomly selecting three fields of view in the brain slice injury lesion area and using Image J software to quantify the fluorescence signal intensity of MAG in the selected fields as the fluorescence intensity of MAG per unit pixel area, and taking the average value. **** represents P<0.0001, n=5.

### Detailed Description of the Invention

### Definitions

As used herein, the term "composition" refers to a formulation suitable for administration to an intended animal subject for therapeutic purposes, which comprises at least one pharmaceutically active component, such as a compound. Optionally, the composition further comprises at least one pharmaceutically acceptable carrier or excipient.

The term "pharmaceutically acceptable" means that the substance does not have the property that, considering the disease or condition to be treated and the respective route of administration, will allow rational and prudent medical practitioners to avoid administering the substance to the patient. For example, for injectables, it is often required that such substance is substantially sterile.

As used herein, the terms "prophylactically effective amount" and "therapeutically effective amount" mean that the substance and the amount of the substance are effective to prevent, alleviate or ameliorate one or more symptoms of a disease or condition, and/or prolong the survival of the subject receiving the treatment.

As used herein, "treatment" includes the administration of a compound of the present application or a pharmaceutically acceptable salt thereof to alleviate the symptoms or complications of a disease or condition, or to eliminate the disease or condition. The term "alleviate" as used herein is used to describe the process of reducing the severity of signs or symptoms of a disorder. Symptoms can be alleviated but not eliminated. In one embodiment, administration of the pharmaceutical composition of the present application results in the elimination of signs or symptoms.

As used herein, "prevention" or "preventing" includes administration of a compound of the present application, or a pharmaceutically acceptable salt thereof, to prevent or stop the development of a particular disease, symptom, or complication.

As used herein, the term "C₁₋₁₀" or "C₃₋₁₀" or similar expressions means having 1 to 10 or 3 to 10 carbon atoms. For example, C₁₋₁₀ alkyl refers to an alkyl group having 1 to 10 carbon atoms, such as methyl, ethyl, propyl, isopropyl, butyl, isobutyl, pentyl, decyl, and the like.

### Compound or a pharmaceutically acceptable salt thereof

Compounds that are applicable in the methods or uses of the present invention include a compound of formula I: 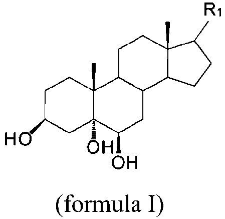
or a pharmaceutically acceptable salt thereof,
wherein R₁ is selected from H, -CN, fluoro, chloro, C₁₋₁₀ alkyl, fluoro or chloro substituted C₁₋₁₀ alkyl, C₁₋₁₀ alkoxy, fluoro or chloro substituted C₁₋₁₀ alkoxy, and C₃₋₁₀ cycloalkyl. The compound of formula I, or a pharmaceutically acceptable salt thereof, is also referred to herein as "compound(s) of the present invention/application".

In one embodiment, R₁ is H, and the compound is 5α-androst-3β,5,6β-triol (sometimes referred to as "YC-6" in the present invention), which has the structure of formula (II):

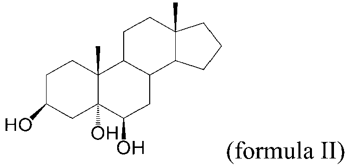

In one embodiment, R₁ is -CHCH₂CH₃, and the compound is 17-propylene-androst-3β,5α,6β-triol. In one embodiment, R₁ is -CH(CH₃)₂, and the compound is 17-isopropyl-androst-3β,5α,6β-triol. In one embodiment, R₁ is -CH(CH₂)₃CH₃, and the compound is 17-butyl-androst-3β,5α,6β-triol. In one embodiment, R₁ is -CH(CH₃)(CH₂)₃CH(CH₃)₂, and the compound is cholestane-3β, 5α, 6β-triol.

The compounds of the present invention can be formulated in the form of pharmaceutically acceptable salts. The expected pharmaceutically acceptable salt forms include, but are not limited to, mono-, di-, tri-, and tetra-salts. Pharmaceutically acceptable salts are non-toxic at the amount and concentration to which they are administered. The preparation of such salts can facilitate pharmacological uses by changing the physical properties of the compound without preventing it from exerting physiological effects. Useful changes in physical properties include lowering the melting point to facilitate transmucosal administration, and increasing solubility to facilitate administration of higher concentrations of drugs.

Pharmaceutically acceptable salts include acid addition salts, such as those containing sulfate, chloride, hydrochloride, fumarate, maleate, phosphate, sulfamate, acetate, citrate, lactate, tartrate, methanesulfonate, ethanesulfonate, benzenesulfonate, p-toluenesulfonate, cyclohexylsulfamate and quinate salts. Pharmaceutically acceptable salts can be obtained from acids such as hydrochloric acid, maleic acid, sulfuric acid, phosphoric acid, sulfamic acid, acetic acid, citric acid, lactic acid, tartaric acid, malonic acid, methanesulfonic acid, ethanesulfonic acid, benzenesulfonic acid, p-toluenesulfonic acid, cyclohexylsulfamic acid, fumaric acid and quinic acid.

When acidic functional groups such as carboxylic acids or phenols are present, pharmaceutically acceptable salts also include base addition salts, such as those containing benzathine penicillin, chloroprocaine, choline, diethanolamine, ethanolamine, tert-butylamine, ethylenediamine, meglumine, procaine, aluminum, calcium, lithium, magnesium, potassium, sodium, ammonium, alkylamine and zinc. Such salts can be prepared using appropriate corresponding bases.

Pharmaceutically acceptable salts can be prepared through standard techniques. For example, the compound in its free base form can be dissolved in a suitable solvent, such as an aqueous solution or a water-alcohol solution containing a suitable acid, and then the solution is evaporated for separation. In another example, the salt is prepared by reacting the free base with an acid in an organic solvent.

Thus, for example, if the specific compound is a base, the desired pharmaceutically acceptable salt can be prepared by any suitable method available in the art, for example, by treating the free base with an inorganic acid such as hydrochloric acid, hydrobromic acid, sulfuric acid, nitric acid, phosphoric, or the like, or an organic acid such as acetic acid, maleic acid, succinic acid, mandelic acid, fumaric acid, malonic acid, pyruvic acid, oxalic acid, glycolic acid, salicylic acid, pyranosidyl acid such as glucuronic acid or galacturonic acid, α-hydroxy acid such as citric acid or tartaric acid, amino acid such as aspartic acid or glutamic acid, aromatic acid such as benzoic acid or cinnamic acid, sulfonic acid such as p-toluenesulfonic acid or ethanesulfonic acid or the like.

Similarly, if the specific compound is an acid, the desired pharmaceutically acceptable salt can be prepared by any suitable method, for example, by treating the free acid with an inorganic base or an organic base, such as an amine (primary, secondary or tertiary amine), alkali metal hydroxides or alkaline earth metal hydroxides or the like. Illustrative examples of suitable salts include organic salts derived from amino acids (such as L-glycine, L-lysine, and L-arginine), ammonia, primary, secondary, and tertiary amines, and cyclic amines (such as hydroxyethylpyrrolidine, piperidine, morpholine and piperazine), and inorganic salts derived from sodium, calcium, potassium, magnesium, manganese, iron, copper, zinc, aluminum and lithium.

The pharmaceutically acceptable salts of the compounds can exist as complexes. Examples of complexes include 8-chlorotheophylline complexes (such as, for example, diphenhydramate: diphenhydramine 8-chlorotheophylline (1:1) complex; Dramamine) and various complexes comprising cyclodextrin.

### Pharmaceutical composition

Another aspect of the present invention provides a pharmaceutical composition comprising a therapeutically or prophylactically effective amount of a compound of formula I (for example, YC-6) or a pharmaceutically acceptable salt thereof, and a pharmaceutically acceptable carrier.

In the present invention, "pharmaceutical composition" refers to a composition comprising a compound of formula I and a pharmaceutically acceptable carrier, wherein the compound and the pharmaceutically acceptable carrier are present in the composition in a mixed form. The composition will generally be used in the treatment of human subjects. However, they can also be used to treat similar or same conditions in other animal subjects. In this context, the terms "subject", "animal subject" and similar terms refer to human and non-human vertebrates, such as mammals, such as non-human primates, competitive animals and commercial animals, such as horses, cattle, pigs, sheep, rodents, and pets (such as dogs and cats).

The appropriate dosage form depends in part on the use or route of administration, for example, it can be oral, transdermal, transmucosal, by inhalation or by injection (parenteral). Such dosage forms should enable the compound to reach target cells. Other factors are well known in the art and include considerations such as toxicity and dosage forms that delay the compound or composition from exerting its effects.

Carriers or excipients can be used to produce the composition. The carriers or excipients can be selected to facilitate administration of the compound. Examples of carriers include calcium carbonate, calcium phosphate, various sugars (such as lactose, glucose, or sucrose), or starch types, cellulose derivatives, gelatin, vegetable oils, polyethylene glycol, and physiologically compatible solvents. Examples of physiologically compatible solvents include sterile water for injection (WFI) solutions, saline solutions, and glucose.

The composition or components of the composition can be administered by different routes, including intravenous, intraperitoneal, subcutaneous, intramuscular, oral, transmucosal, rectal, transdermal, or inhalation routes. In some embodiments, injections or lyophilized powder injections are preferred. For oral administration, for example, the compound may be formulated into conventional oral dosage forms such as capsules and tablets, and liquid preparations such as syrups, elixirs and concentrated drops.

Pharmaceutical preparations for oral use can be obtained, for example, by combining the composition or its components with solid excipients, optionally grinding the resulting mixture, and treating the mixture of particles after adding suitable adjuvants (if necessary), thereby obtaining tablets or dragees. Suitable excipients are, in particular, fillers such as sugars including lactose, sucrose, mannitol or sorbitol; cellulose preparations such as corn starch, wheat starch, rice starch, potato starch, gelatin, tragacanth, methyl cellulose, hydroxypropyl methyl cellulose, sodium carboxymethyl cellulose (CMC) and/or polyvinylpyrrolidone (PVP: povidone). If desired, disintegrating agents may be added, such as cross-linked polyvinylpyrrolidone, agar, or alginic acid or their salts, such as sodium alginate.

Alternatively, injections (parenteral administration) can be used, for example intramuscular, intravenous, intraperitoneal and/or subcutaneous injection. For injection, the composition of the invention or its components are formulated as a sterile liquid solution, preferably in a physiologically compatible buffer or solution, such as saline solution, Hank's solution or Ringer's solution. In addition, the composition or its components can be formulated in a solid form and re-dissolved or suspended immediately before use. It can also be produced in the form of lyophilized powder.

Administration can also be by transmucosal, topical or transdermal means. For transmucosal, topical or transdermal administration, penetrants suitable for the barrier to be penetrated are used in the formulation. Such penetrants are generally known in the art and include, for example, for transmucosal administration, bile salts and fusidic acid derivatives. In addition, detergents can be used to promote penetration. Transmucosal administration, for example, can be by nasal spray or suppository.

The effective amount of various components to be administered can be determined by standard procedures, taking into account factors such as IC₅₀ of the compound, the biological half-life of the compound, the age, size, and body weight of the subject, and the conditions associated with the subject. The importance of these and other factors is well known to those of ordinary skill in the art. Generally, the dosage will be between about 0.01 mg/kg and 50 mg/kg of the subject to be treated, preferably between 0.1 mg/kg and 20 mg/kg. Multiple doses can be used.

The composition of the present invention or its components can also be used in combination with other therapeutic agents for treating the same disease. Such combined use includes administration of these compounds and one or more other therapeutic agents at different times, or simultaneous use of these compounds and one or more other therapeutic agents. In some embodiments, the dosage of one or more compounds of the invention or other therapeutic agents used in combination can be modified, for example, by methods known to those of skill in the art to reduce the dose relative to the compound or therapeutic agent used alone.

It is to be understood that the combined use or combination includes use with other therapies, drugs, medical procedures, etc., wherein the other therapies or procedures may be administered at a time different from the composition of the present invention or its components (eg, in a short period of time (such as a few hours, such as 1, 2, 3, 4-24 hours) or in a longer period of time (such as 1-2 days, 2-4 days, 4-7 days, 1-4 weeks)) or at the same time as the composition of the invention or its components. The combined use also includes use with one-time or infrequently administered therapies or medical procedures (such as a surgery), accompanied by administration of the composition of the invention or its components within a short period of time or a longer period of time before or after the other therapies or procedures. In some embodiments, the present invention is used to deliver the composition of the present invention or its components and one or more other pharmaceutical therapeutic agents, and they are delivered by same or different routes.

The combined administration of any route of administration includes the delivery of the composition of the invention or its components and one or more other pharmaceutical therapeutic agents in any formulation by the same route of administration, including formulations in which the two compounds are chemically linked and retain their respective therapeutic activity when administered. In one aspect, the other drug therapy can be coadministered with the composition of the invention or its components. The combined use by co-administration includes the administration of the co-formulation or chemically linked compounds, or the administration in a short period of time (eg, within one hour, within 2 hours, within 3 hours, up to within 24 hours) of two or more compounds in independent formulations, which are administered by the same or different routes.

Co-administration of independent formulations includes co-administration by delivery via one device, such as the same inhalation device, the same syringe, etc., or administration by different devices within a short period of time relative to each other. A co-formulation of a compound of the present invention and one or more additional pharmacotherapies delivered by the same route of administration includes preparing the materials together so that they can be administered by one device, including combining different compounds in one formulation, or modifying compounds so that they are chemically linked together but still retain their biological activity. Such chemically linked compounds may include a linker that separates the two active ingredients, where the linker is substantially maintained in the body, or may be degraded in the body.

### Medical use and method of treatment

In one aspect, the present invention provides use of any compound of formula I or a pharmaceutically acceptable salt thereof in the manufacture of a medicament for preventing or treating a radiation-induced brain injury in a subject.

In another aspect, the present invention provides a method for preventing or treating a radiation-induced brain injury in a subject, comprising administering a therapeutic effective amount of any compound of formula I or a pharmaceutically acceptable salt thereof, or a pharmaceutical composition comprising any compound of formula I or a pharmaceutically acceptable salt thereof, to the subject in need thereof.

In yet another aspect, the present invention provides any compound of formula I or a pharmaceutically acceptable salt thereof, or a pharmaceutical composition comprising any compound of formula I or a pharmaceutically acceptable salt thereof, for use in preventing or treating a radiation-induced brain injury.

In any one of the above aspects, in a preferred embodiment, the compound is YC-6.

In any one of the above aspects, in a preferred embodiment, the radiation dose for the radiation-induced brain injury is 50 Gy or more, such as 50 Gy to 100 Gy, such as 50 Gy to 90 Gy, 50 Gy to 80 Gy, 50 Gy to 70 Gy, 50 Gy to 60 Gy, 60 Gy to 100 Gy, 60 Gy to 90 Gy, 60 Gy to 80 Gy, 60 Gy to 70 Gy, 70 Gy to 100 Gy, 70 Gy to 90 Gy, 70 Gy to 80 Gy, 80 Gy to 100 Gy, 80 Gy to 90 Gy, or 90 Gy to 100 Gy. In any one of the above aspects, in a preferred embodiment, the radiation dose for the radiation-induced brain injury is 100 Gy or less. For example, the radiation dose for radiation-induced brain injury is 50 Gy, 55 Gy, 60 Gy, 65 Gy, 70 Gy, 75 Gy, 80 Gy, 85 Gy, 90 Gy, 95 Gy, or 100 Gy. In other embodiments, the radiation dose for the radiation-induced brain injury is lower than 50 Gy. In other embodiments, the radiation dose for the radiation-induced brain injury is higher than 100 Gy.

In any one of the above aspects, in a preferred embodiment, the radiation-induced brain injury is a gamma ray radiation-induced brain injury, such as cobalt-60 gamma ray. In any one of the above aspects, in other embodiments, the radiation-induced brain injury is a radiation-induced brain injury caused by alpha rays, beta rays, gamma rays, X-rays, neutrons, electron beams, proton beams, particle beams, and combinations thereof. Ordinary X-ray machines are used to treat superficial tumors. The high-energy X generated by various accelerators can treat tumors in any part of the body, and the treatment effect is particularly good for deeper tumors.

In any one of the above aspects, the radiation-induced brain injury is an acute, early-delayed, or late-delayed radiation-induced brain injury, and more preferably, a late-delayed radiation-induced brain injury. According to imaging findings and characteristics, the late-delayed type can be divided into no lesion stage, edema stage, necrosis stage, and cystic stage, and each stage may appear simultaneously or sequentially in different parts of the same patient's brain. (1) Non lesion stage: The patient has no visible lesions on imaging, but has clinical manifestations of brain damage, including headaches, cognitive impairment, seizures, neurological dysfunction (such as limb numbness), and other new symptoms of brain damage after radiotherapy. (2) Edema stage: Head imaging examination reveals that brain injury lesions are mainly characterized by white matter edema with blurred boundaries. (3) Necrosis stage: Local necrosis of brain tissue lesions may occur, accompanied by bleeding or exudation. Head MRI shows uneven signal, and enhanced scanning shows enhancement. (4) Cystic stage: The patient's head MRI shows clear boundaries and cystic changes of the radiation-induced brain injury lesion, with signals close to free water signals and with or without space occupying effects. The cystic stage lesions may remain stable for a long time, but they may also increase rapidly, causing cerebral herniation and leading to decreased consciousness, coma, and even death in patients. In a preferred embodiment, the radiation-induced brain injury is the edema stage or necrosis stage of a late-delayed radiation-induced brain injury.

Therefore, in some preferred embodiments of the present invention, the radiation-induced brain injury is a 50 Gy or higher gamma ray radiation-induced brain injury, such as a 50 Gy or 60 Gy gamma ray radiation-induced brain injury. In some preferred embodiments of the present invention, the radiation-induced brain injury is a gamma ray early-delayed or late-delayed radiation-induced brain injury, such as a gamma ray late-delayed radiation-induced brain injury. In some preferred embodiments of the present invention, the radiation-induced brain injury is the edema stage or necrosis stage of a gamma ray late-delayed radiation-induced brain injury.

In any one of the above aspects, the subject has a tumor, such as a neurological tumor (e.g. brain tumor, medulloblastoma, neuroblastoma, and pituitary tumor) or a head and neck tumor. The term "head and neck tumor" includes three major parts based on different locations: neck tumor, otolaryngological tumor, and oral and maxillofacial tumor. Neck tumor includes, for example, thyroid tumor; Otolaryngological tumor includes, for example, nasopharyngeal cancer, laryngeal cancer, paranasal sinus cancer, etc; Oral and maxillofacial tumor includes, for example, oral cancers such as tongue cancer, gingival cancer, cheek cancer, maxillary sinus cancer, and tonsillar cancer, etc.

In some embodiments, the present invention provides a method for preventing or treating a radiation-induced brain injury, particularly early-delayed or late-delayed radiation-induced brain injury, comprising administering any compound of formula I or a pharmaceutically acceptable salt thereof to a subject who has undergone a radiation therapy, wherein the administration is initiated immediately after the radiation therapy and ended before the occurrence of the radiation-induced brain injury.

In some embodiments, the present invention provides a method for preventing or treating an acute radiation-induced brain injury, comprising administering a compound of formula I of the present invention (e.g. YC-6) or a pharmaceutically acceptable salt thereof to a subject who has undergone a radiation therapy, wherein the administration is initiated immediately after the radiation therapy and ended before the occurrence of the acute radiation-induced brain injury. For example, the administration is initiated immediately after the radiation therapy and lasts for no more than 10 days. For example, the administration is initiated on the day after the radiation therapy and lasts for 1 to 10 days, such as 1 to 7 days, 1 to 5 days, 1 to 3 days, 3 to 10 days, 3 to 7 days, 3 to 5 days, 5 to 10 days, or 5 to 7 days. For example, the administration may be initiated on the day after the radiation therapy and last for 1, 2, 3, 4, 5, 6, 7, 8, 9, or 10 days.

In some embodiments, the present invention provides a method for preventing or treating an early-delayed radiation-induced brain injury, comprising administering a compound of formula I (e.g. YC-6) or a pharmaceutically acceptable salt thereof to a subject who has undergone a radiation therapy, wherein the administration is initiated immediately after the radiation therapy and ended before the occurrence of the early-delayed radiation-induced brain injury. For example, the administration is initiated immediately after the radiation therapy and lasts for less than one month. For example, the administration is initiated on the day after the radiation therapy and lasts for 1 to 30 days, such as 5 to 30 days, 10 to 30 days, 10 to 25 days, 5 to 25 days, 5 to 20 days, 10 to 15 days, 15 to 20 days, or 10 to 20 days. For example, the administration may be initiated on the day after the radiation therapy and last for 1, 3, 5, 6, 7, 8, 9, 10, 11, 12, 13, 14, 15, 16, 17, 18, 19, 20, 21, 22, 23, 24, 25, 26, 27, 28, 29, or 30 days.

In some embodiments, the present invention provides a method for preventing or treating a late-delayed radiation-induced brain injury, comprising administering a compound of formula I of the present invention (e.g. YC-6) or a pharmaceutically acceptable salt thereof to a subject who has undergone a radiation therapy, wherein the administration is initiated immediately after the radiation therapy and ended before the occurrence of the late-delayed radiation-induced brain injury. For example, the administration is initiated immediately after the radiation therapy and lasts for less than 6 months. For example, the administration is initiated on the day after the radiation therapy and lasts for 5 to 180 days, such as 5 to 150 days, 5 to 120 days, 5 to 90 days, 5 to 60 days, 5 to 30 days, 10 to 150 days, 10 to 120 days, 10 to 90 days, 10 to 60 days, 10 to 30 days, 15 to 150 days, 15 to 120 days, 15 to 90 days, 15 to 60 days, 15 to 30 days, 20 to 150 days, 20 to 120 days, 20 to 90 days, 20 to 60 days, 20 to 30 days, 5 to 30 days, 10 to 30 days, 10 to 25 days, 5 to 25 days, 5 to 20 days, 10 to 15 days, 15 to 20 days or 10 to 20 days. For example, the administration may be initiated on the day after the radiation therapy and last for 1, 3, 5, 6, 7, 8, 9, 10, 11, 12, 13, 14, 15, 16, 17, 18, 19, 20, 21, 22, 23, 24, 25, 26, 27, 28, 29, 30, 45, 60, 75, 90, 105, 120, 150, or 180 days.

### EXAMPLES

Using gamma knife irradiation induced focal radiation-induced brain injury in C57BL/6 mice as a model, the preventive or therapeutic effects of YC-6 on BRI were studied at the animal level. The experimental results show that prophylactic administration of 60mg/kg/d of YC-6 for 2 weeks, followed by discontinuation until week 8, significantly reduced the volume of brain lesions, indicating a "post protective" effect of YC-6 short-term administration. Moreover, a full 8-week YC-6 administration showed a trend towards further reducing the volume of brain lesions, reducing the damage caused by gamma ray radiation to brain tissue, without affecting body weight. YC-6 reduced the volume of RBI lesions, alleviated the injury caused by gamma ray radiation to brain tissue, and has a good preventive or therapeutic effect on RBI.

### Experimental method

Animal grouping: Animals were randomly divided into four groups based on body weight, as shown in Table 1. The experimental protocol is shown in Fig. 1.

**Table 1. Animal grouping and treatment methods**

| **Experimental group** | **n** | **Treatment** |
|---|---|---|
| Normal control group Control+Saline | 5 | Not being subjected to gamma ray radiation, intraperitoneal injection of physiological saline twice a day, with an interval of 6-8 hours, for 8 consecutive weeks, with a dosage volume of 10ml/kg; |
| Radiation-induced brain injury model group RBI+Saline | 5 | Construction of gamma ray radiation model, intraperitoneal injection of physiological saline twice a day, with an interval of 6-8 hours, for 8 consecutive weeks, with a dosage volume of 10ml/kg; |
| YC-6 treatment 2-week group RBI+YC-6(60mg/kg/d-2W) | 5 | Within 10 minutes after constructing gamma ray radiation model, 6mg/ml of YC-6 was injected via the tail vein. Starting from the second day, 3mg/ml of YC-6 was injected via the tail vein twice a day, with an interval of 6-8 hours. After continuous administration for 2 weeks, the administration was stopped for observation for 6 weeks. The dosage volume was 10ml/kg; |
| YC-6 treatment 8-week group RBI+YC-6(60mg/kg/d-8W) | 5 | Within 10 minutes after constructing gamma ray radiation model, 6mg/ml of YC-6 was injected via the tail vein. Starting from the second day, 3mg/ml of YC-6 was injected via the tail vein twice a day, with an interval of 6-8 hours. After continuous administration for 2 weeks, 3mg/ml of YC-6 30mg/kg was injected intraperitoneally twice a day, with an interval of 6-8 hours, for 6 consecutive weeks. The dosage volume was 10ml/kg. |

### Model constructing method for focal radiation-induced brain injury caused by gamma ray radiation

The model constructing method for focal radiation-induced brain injury caused by gamma ray radiation was modified based on reported literature. After anesthesia of mouse by intraperitoneal injection of 0.1% pentobarbital sodium solution, the body was fixed on a customized platform, and the mouse skull was scanned by X-ray computed tomography scanner for imaging processing (radiation energy of 80kV, imaging range of 250mm, layer thickness and interlayer spacing of 0.625mm, pixel size of 0.49*0.49mm). The animal irradiation position and gamma knife irradiation depth were determined based on the scanning imaging results. The mouse and customized platform were placed in a stereotactic gamma ray irradiation system for irradiation, with the irradiation site in the left thalamus of the mouse. The coordinates of the irradiation center positioning point were: 2mm behind the anterior fontanelle (AP), 2mm left of the midline (L), and 2mm below the skull dura mater (dorsal ventral DV). The irradiation dose was 50 Gy at the 50% isodose line, and the irradiation rate was approximately 1.7 Gy/min. Within 10 minutes after model construction, the treatment group was injected with 6mg/ml of YC-6 via tail vein, with a dosage volume of 10ml/kg.

### Animal weighting

Weighing was performed once a week before and after the gamma ray radiation model construction for 8 weeks.

### Quantitative analysis of brain lesion volume using magnetic resonance imaging (MRI)

After 8 weeks of model construction with gamma ray radiation, mice were induced to rapid anesthesia with 3% isoflurane and maintained under anesthesia with 2% isoflurane. Prior to detection, each mouse was injected with 200 µl of contrast agent solution at a concentration of approximately 48mg/ml. Then, the brain of the mice was subjected to a T1 weighted sequence scanning using a 7-T Brooke small animal magnetic resonance imaging (MRI) machine. The high signal area in the T1 sequence scan image represented the area of cerebral edema lesions. The obtained T1 weighted sequence dicom format images were imported into the ITK-SNAP software. Following the software's instructions, all high signal focal areas of the gamma knife irradiation area were extracted from the entire brain, especially the left (radiated) hemisphere in several consecutive image slices. By quickly and finely adjusting the contrast (gradually adjusting the threshold) and loading segmented image files step by step, all data on brain necrosis volume was recorded and analyzed, and the volume of the lesion was calculated.

### Animal brain tissue sampling and embedding of slices

After MRI imaging detection, animals were rapidly anesthetized with 3% isoflurane and placed on an dissecting table in a supine position. The skin was cut open and the chest cavity was exposed. About 30ml of physiological saline per animal was perfused into the heart, and the blood in the blood vessels was washed away. The intact brain tissue was taken out by decapitation and fixed in a 4% paraformaldehyde solution. After fixation for 24 hours, dehydration was performed with 30% sucrose for 48 hours. the cerebellum, olfactory bulb, and part of the forebrain were removed. The remaining brain tissue was selected for OTC embedding, and the slices were frozen, with a thickness of the brain tissue slices being 20 µm, for subsequent immunofluorescence staining.

### Immunofluorescence staining of GFAP, CC1, and MAG proteins in brain slices

Brain tissue slices were left at room temperature for 15 minutes. After the room temperature was restored, they were soaked in PBS for 10 minutes to remove OCT on the slices. A chemical oil pen was used to draw circles along the brain tissue to be stained;
After blocking the brain slices with a blocking solution composed of 1% BSA and 0.25% Triton X-100 at room temperature for 1 hour, the brain slices were incubated overnight with primary antibodies in a refrigerator at 4 °C. The dilution ratio of MAP2 antibody was 1:500, GFAP antibody was 1:500, Iba1 antibody was 1:1000, CD68 antibody was 1:100, CC1 antibody was 1:1000, and MAG antibody was 1:500;
The brain slices were washed three times with PBS, each time for 10 minutes;
Fluorescent secondary antibodies for the corresponding species were added, followed by incubation at 37 °C in the dark for 1 hour. The dilution ratio of fluorescent secondary antibodies was 1:1000;
Washing was performed three times with PBS, each time for 10 minutes;
DAPI was added for counterstaining, followed by incubation at room temperature in the dark for 3-5 minutes;
Washing was performed three times with PBS, each time for 10 minutes, and the slices were sealed with a sealing agent.

The brain slices stained with immunofluorescence were imaged using a confocal laser microscope, and images of the same size were taken for each slice; Image J software was used to analyze the images, and after background subtraction, all images were set with the same threshold setting. The fields of view of the injury lesion area in the brain slices were selected, and the number of GFAP positive cells, CC-1 positive cells, and fluorescence intensity of MAG per unit area were analyzed with the Analyze Particles function in Image J.

### Data statistical methods

The data was displayed as mean ± standard error (Mean ± SEM). Graphpad Prism 7.0 software was used for statistical analysis and plotting of the data. If the data satisfied normality and homogeneity of variance, One-Way ANOVA would be used, and Tukey's method would be used for inter group comparison. If the data did not satisfy normality or homogeneity of variance, non-parametric tests (Kruskal-Wallis rank sum test) would be used, and Uncorrected Dunn's test would be used for inter group comparison. The weight changes of each group of animals at different time points were analyzed using Two Way ANOVA, and the comparison between groups was conducted using Tukey's multiple comparisons test.

### Example 1: YC-6 significantly reduced the volume of radiation-induced brain injury induced by gamma ray

This study observed that after 8 weeks of construction of gamma ray radiation model, MRI images of the mouse brain showed severe lesions (white signal area) in the brain tissue of the gamma knife irradiated area. On the day of gamma knife radiation model constructing, YC-6 was given preventively. MRI imaging results showed that YC-6 was only given for 2 weeks and stopped at week 8, significantly reducing lesion volume, indicating that short-term administration of YC-6 has a "post protective" effect. The full 8-week administration of YC-6 showed a better trend in reducing lesion volume compared to short-term administration, indicating that YC-6 can alleviate the injury caused by gamma ray irradiation to brain tissue. The results are shown in Table 2 and Figs. 2A and 2B.

**Table 2. YC-6 significantly reduced the volume of brain lesions (Mean+SEM)**

| **Group** | **n** | **Volume of lesion (mm³)** |
|---|---|---|
| RBI+Saline model group | 5 | 15.28 ± 0.98 |
| RBI+YC-6(60mg/kg/d-2W) group | 5 | 12.28 ± 0.63^{#} |
| RBI+YC-6(60mg/kg/d-8W) group | 5 | 10.27 ± 1.50^{#} |

| | | |
|---|---|---|
| *Note: Compared with the RBI+Saline model group, # represents P<0.05.* | | |

### Example 2. YC-6 inhibited the activation of astrocytes after radiation-induced brain injury

Glial fibroblast acidic protein (GFAP) is a marker of astrocyte activation; The proliferation of astrocytes is often accompanied by an increase in GFAP expression, therefore GFAP is often used as a biomarker for astrocyte proliferation. When the central nervous system is damaged, astrocytes are in an "activated" state, with changes in their morphology, quantity, and biological function, transforming from benign "resting astrocytes" to "reactive astrocytes".

This study found that after 8 weeks of gamma knife radiation, astrocytes in the thalamus of the lesion were significantly activated, manifested by upregulation of GFAP expression, increased number of astrocytes, cell hypertrophy, and the emission of many long and branched protrusions from the cell body. YC-6 administration for 8 weeks significantly inhibited the activation of astrocytes. The results are shown in Table 3 and Figs. 3A and 3B.

**Table 3. Number of GFAP positive astrocytes (Mean±SEM)**

| **Group** | **n** | **Number of astrocytes (Astrocytes/mm²)** |
|---|---|---|
| Control+ Saline normal group | 5 | 57.80 ± **2.69** |
| RBI + Saline model group | 5 | 558.20 ± **10.07****** |
| RBI+YC-6 (60mg/kg/d-8W) group | 5 | 357.00 ± **10.61^{####}** |

| | | |
|---|---|---|
| *Note: Compared with the Control+Saline normal group, **** represents P<0.0001; Compared with the RBI+Saline model group, ^{####} represents P<0.0001.* | | |

### Example 3: YC-6 inhibited the reduction of oligodendrocytes and restored MAG level

Oligodendrocytes are formed by the proliferation and differentiation of precursor cells of oligodendrocytes. Their main function is to wrap around the axons of neurons, form an insulating myelin sheath structure, assist in the rapid transmission of bioelectric signals, and maintain and protect the normal function of neurons. Interruption of their proliferation and differentiation will lead to damage to the central nervous system. CC-1 (APC) is a biomarker of mature oligodendrocytes. Myelin associated glycoprotein (MAG) is expressed by oligodendrocytes and Schwann cells in the peripheral nervous system, and is located in the innermost layer of the myelin sheath in direct contact with axons. It participates in the formation and maintenance of myelin sheath integrity by mediating the interaction between glial cells and axons.

After 8 weeks of gamma knife radiation, the number of mature oligodendrocytes in the thalamus of the lesion significantly decreased, manifested as a decrease in CC1 immunofluorescence staining signal. Meanwhile, the MAG level decreased, indicating a pathological change in the myelin sheath structure of the posterior process after BRI. YC-6 administration for 8 weeks significantly inhibited the reduction of oligodendrocytes and restored MAG level, protecting myelin sheaths. The results are shown in Tables 4 and 5 and Figs. 4A and 4B.

**Table 4. Number of CC1 positive oligodendrocytes (Mean±SEM)**

| **Group** | **n** | **Number of mature oligodendrocytes (Oligodendrocytes/mm²)** |
|---|---|---|
| Control+ Saline normal group | 5 | 1608.00±47.92 |
| RBI + Saline model group | 5 | 200.60±21.47**** |
| RBI+YC-6 (60mg/kg/d-8W) group | 5 | 506.40±30.48^{####} |

| | | |
|---|---|---|
| *Note: Compared with the Control+Saline normal group, **** represents P<0.0001; Compared with the RBI+Saline model group, ^{####} represents P<0.0001.* | | |

**Table 5. MAG fluorescence signal intensity (Mean±SEM)**

| **Group** | **n** | **MAG fluorescence signal intensity** |
|---|---|---|
| Control+ Saline group | 5 | 1.04±0.047 |
| RBI + Saline group | 5 | 0.31±0.018**** |
| RBI+YC-6 (60mg/kg/dg-8W) group | 5 | 0.44±0.018^{####} |

| | | |
|---|---|---|
| *Note: Compared with the Control+Saline normal group, **** represents P<0.0001; Compared with the RBI+Saline model group, ^{####} represents P<0.0001.* | | |

## Claims

1. Use of a compound of formula I: 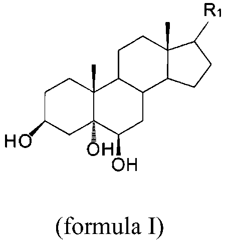
or a pharmaceutically acceptable salt thereof in the manufacture of a medicament for preventing or treating a radiation-induced brain injury,
wherein R₁ is selected from H, -CN, fluoro, chloro, C₁₋₁₀ alkyl, fluoro or chloro substituted C₁₋₁₀ alkyl, C₁₋₁₀ alkoxy, fluoro or chloro substituted C₁₋₁₀ alkoxy, and C₃₋₁₀ cycloalkyl.

2. The use of claim 1, wherein the radiation dose for the radiation-induced brain injury is 50 Gy or more.

3. The use of claim 1 or 2, wherein the radiation-induced brain injury is gamma ray radiation-induced brain injury.

4. The use of any one of claims 1 to 3, wherein the radiation-induced brain injury is an early-delayed radiation-induced brain injury or a late-delayed radiation-induced brain injury.

5. The use of any one of claims 1 to 3, wherein the radiation-induced brain injury is an edema stage or a necrosis stage of a late-delayed radiation-induced brain injury.

6. The use of claim 1, wherein R₁ is H, -CHCH₂CH₃, -CH(CH₃)₂, -CH(CH₂)₃CH₃, or - CH(CH₃)(CH₂)₃CH(CH₃)₂.

7. The use of claim 1, wherein R₁ is H.
